# EUROPEAN PATENT APPLICATION

(11) **EP 3 263 007 A1**
(43) Date of publication of application: **03.01.2018**
(21) Application number: 16857226.1
(22) Date of filing: 23.09.2016
(51) Int. Cl.: A61B 1/00, A61B 1/04, G02B 23/24, G02B 23/26

(54) **ENDOSCOPE SYSTEM**

(30) Priority: 22.10.2015 JP 2015208029
(71) Applicant: Olympus Corporation, Hachioji-shi, Tokyo 192-8507 (JP)
(72) Inventor: TAKEUCHI, Yuichi, Hachioji-shi Tokyo 192-8507 (JP); WATANABE, Toshiaki, Hachioji-shi Tokyo 192-8507 (JP)
(74) Representative: Gunzelmann, Rainer
(86) International application number: PCT/JP2016/077951
(87) International publication number: WO 2017/068908

(57) **Abstract**

An endoscope system includes a light source apparatus capable of supplying excitation light for generating fluorescence and reference light in a different wavelength band from the fluorescence, an objective optical system, an optical filter configured to block reflected light of the excitation light, an image pickup section configured to separate, and pick up, light that is emitted through the optical filter into the fluorescence, light in a first wavelength band, and light in a second wavelength band, an observation image generation section configured to generate an observation image by using first to third signals obtained by picking up the fluorescence, the light in the first wavelength band, and the light in the second wavelength band, and a control section configured to cause the first signal to be assigned to a green component of a display apparatus, and to cause a signal having a relatively large signal value, between the second and the third signals, to be assigned to a red component of the display apparatus and a signal having a relatively small signal value to be assigned to a blue component of the display apparatus.

## Description

### Technical Field

The present invention relates to an endoscope system, and more particularly, to an endoscope system used for fluorescence observation.

### Background Art

Endoscopic observation in a medical field conventionally adopts fluorescence observation, which is an observation method of determining whether or not a lesion is included at a desired observation position, based on a generation state of fluorescence at the time of irradiation of the desired observation position with excitation light for exciting one of a fluorescent pigment administered to a subject or a predetermined fluorescent substance existing inside a cell of the subject, for example. The fluorescence observation conventionally also uses a method of visualizing a peripheral region of a position where fluorescence is generated, based on reflected light of reference light which is emitted according to radiation of the reference light which is light in a different wavelength band from the fluorescence, for example.

More specifically, for example, Japanese Patent Application Laid-Open Publication No. 2012-23492 discloses an image pickup system configured to generate a combined image of a visible light image that is obtained by picking up reflected light of visible light radiated on a subject having a fluorescent substance and a fluorescent image that is obtained by picking up fluorescence emitted from the fluorescent substance according to radiation of excitation light.

Now, with fluorescence observation, to increase the diagnostic performance at the time of determination of whether a lesion is included at a desired observation position or not, a position where fluorescence is generated and a peripheral region of the position where fluorescence is generated are desirably visualized with color tones allowing the position and the peripheral region to be easily and individually viewed.

However, Japanese Patent Application Laid-Open Publication No. 2012-23492 does not particularly disclose a configuration for allowing a position where fluorescence is generated and a peripheral region of the position where fluorescence is generated to be easily and individually viewed. Therefore, the configuration disclosed in Japanese Patent Application Laid-Open Publication No. 2012-23492 has a problem that the diagnostic performance at the time of diagnosis of a desired observation position is reduced in fluorescence observation.

The present invention has been made in view of the above-described circumstance, and has its object to provide an endoscope system which is capable of increasing the diagnostic performance at the time of diagnosis of a desired observation position in fluorescence observation.

### Disclosure of Invention

### Means for Solving the Problem

An endoscope system according to an aspect of the present invention includes a light source apparatus capable of supplying excitation light for exciting a fluorescent pigment administered to a subject and for generating fluorescence, and reference light that is visible light in a different wavelength band from the fluorescence, an objective optical system, provided in a distal end portion of an insertion section that can be inserted into the subject, to which the fluorescence, reflected light of the reference light generated according to radiation of the reference light, and reflected light of the excitation light generated according to radiation of the excitation light enter as return light, an optical filter including optical characteristics of transmitting the fluorescence and the reflected light of the reference light included in the return light entering from the objective optical system, and of blocking the reflected light of the excitation light included in the return light entering from the objective optical system, an image pickup section configured to be able to separate, and pick up, light that is emitted through the optical filter into the fluorescence, light in a first wavelength band included in the reflected light of the reference light, and light in a second wavelength band, different from the light in the first wavelength band, included in the reflected light of the reference light, an observation image generation section configured to generate an observation image using a first image signal that is obtained by picking up the fluorescence, a second image signal that is obtained by picking up the light in the first wavelength band, and a third image signal that is obtained by picking up the light in the second wavelength band, and to output the observation image to a display apparatus, and a control section configured to control the observation image generation section such that the observation image is generated, by causing the first image signal to be assigned to a first channel corresponding to a green component of the display apparatus, and by causing one image signal having a relatively large signal value, between the second image signal and the third image signal, to be assigned to a second channel corresponding to a red component of the display apparatus and another image signal having a relatively small signal value to be assigned to a third channel corresponding to a blue component of the display apparatus.

An endoscope system according to an aspect of the present invention includes a light source apparatus capable of supplying excitation light for exciting a fluorescent pigment administered to a subject and for generating fluorescence, and reference light that is visible light in a different wavelength band from the fluorescence, an objective optical system, provided in a distal end portion of an insertion section that can be inserted into the subject, to which the fluorescence, reflected light of the reference light generated according to radiation of the reference light, and reflected light of the excitation light generated according to radiation of the excitation light enter as return light, an optical filter including optical characteristics of transmitting the fluorescence and the reflected light of the reference light included in the return light entering from the objective optical system, and of blocking the reflected light of the excitation light included in the return light entering from the objective optical system, an image pickup section configured to be able to separate, and pick up, light that is emitted through the optical filter into the fluorescence, light in a first wavelength band included in the reflected light of the reference light, and light in a second wavelength band, different from the light in the first wavelength band, included in the reflected light of the reference light, an observation image generation section configured to generate an observation image using a first image signal that is obtained by picking up the fluorescence, a second image signal that is obtained by picking up the light in the first wavelength band, and a third image signal that is obtained by picking up the light in the second wavelength band, and to output the observation image to a display apparatus, and a control section configured to control the observation image generation section such that the observation image is generated, by causing the first image signal to be assigned to a first channel corresponding to a green component of the display apparatus, and by causing one image signal having a relatively large signal value, between the second image signal and the third image signal, to be assigned to a second channel corresponding to a red component of the display apparatus and another image signal having a relatively small signal value to be assigned to a third channel corresponding to a blue component of the display apparatus and the first channel.

### Brief Description of the Drawings

Fig. 1 is a diagram showing a configuration of main parts of an endoscope system according to an embodiment;
Fig. 2 is a diagram for describing a specific example of a configuration of the endoscope system according to the embodiment;
Fig. 3 is a diagram showing transmission characteristics as an example of optical characteristics of an excitation light cut filter provided in an endoscope apparatus according to the embodiment;
Fig. 4 is a diagram showing an example of a wavelength band of light emitted from each light source provided in a light source apparatus according to the embodiment, and of a wavelength band of fluorescence that is emitted from a fluorescent pigment;
Fig. 5 is a diagram showing an example configuration of the light source apparatus according to the embodiment, different from the configuration shown in Fig. 2;
Fig. 6 is a diagram showing an example configuration of a rotation filter provided in the light source apparatus in Fig. 5;
Fig. 7 is a diagram showing an example of transmission characteristics of a white light observation filter provided in the rotation filter in Fig. 6; and
Fig. 8 is a diagram showing an example of transmission characteristics of a fluorescence observation filter provided in the rotation filter in Fig. 6.

### Best Mode for Carrying Out the Invention

Hereinafter, an embodiment of the present invention will be described with reference to the drawings.

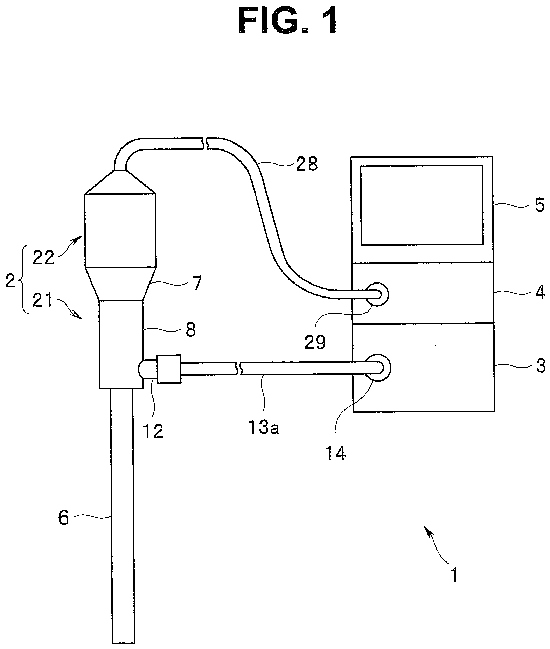

Figs. 1 to 8 relate to an embodiment of the present invention.

As shown in Fig. 1, an endoscope system 1 includes an endoscope apparatus 2 configured to be inserted into a subject, and to output an image signal obtained by picking up an image of a target such as a living tissue inside the subject, a light source apparatus 3 configured to supply, to the endoscope apparatus 2, light which is to be radiated on the target, a video processor 4 configured to generate an observation image or the like by subjecting the image signal outputted from the endoscope apparatus 2 to predetermined image processing, and to output the observation image or the like, and a display apparatus 5 configured to display the observation image or the like outputted from the video processor 4 on a screen. Fig. 1 is a diagram showing a configuration of main parts of the endoscope system according to the embodiment.

The endoscope apparatus 2 is configured to be able to perform observation using fluorescence that is generated according to radiation of excitation light on a fluorescent pigment administered to a subject, and reference light, which is light in a different wavelength band from the fluorescence. Moreover, the endoscope apparatus 2 includes a telescope 21 provided with an elongated insertion section 6, and a camera unit 22, which is detachable from an eyepiece section 7 of the telescope 21.

The telescope 21 includes the elongated insertion section 6, which can be inserted into a subject, a grasping section 8 provided at a proximal end portion of the insertion section 6, and the eyepiece section 7 provided at a proximal end portion of the grasping section 8.

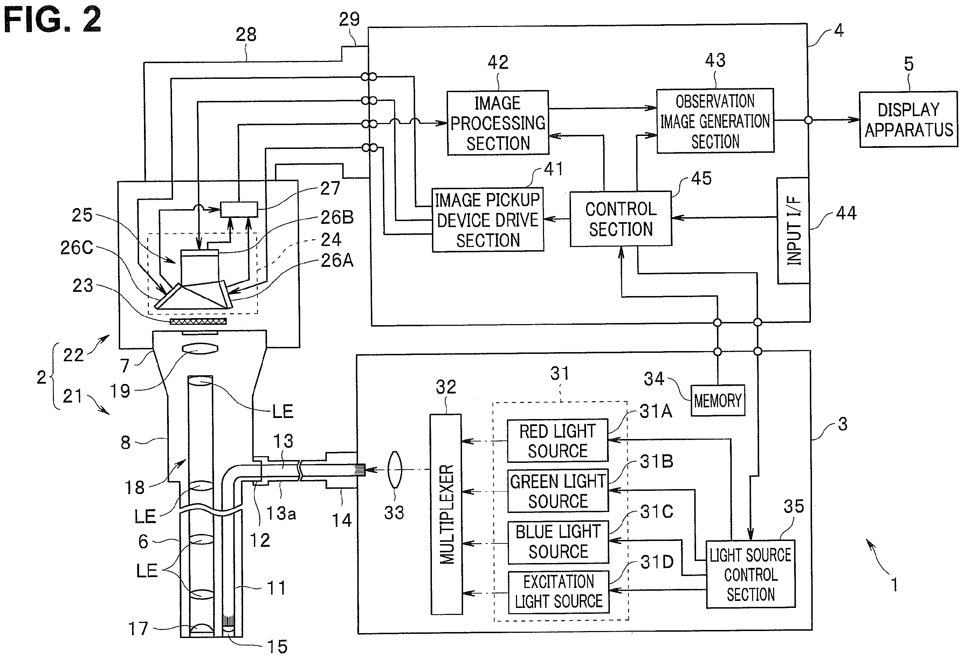

As shown in Fig. 2, a light guide 11 configured to transmit light supplied via a cable 13a is inserted inside the insertion section 6. Fig. 2 is a diagram for describing a specific example of a configuration of the endoscope system according to the embodiment.

As shown in Fig. 2, an emitting end portion of the light guide 11 is disposed near an illumination lens 15 at a distal end portion of the insertion section 6. Also, an incident end portion of the light guide 11 is disposed at a light guide pipe sleeve 12 provided in the grasping section 8.

As shown in Fig. 2, a light guide 13 configured to transmit light supplied from the light source apparatus 3 is inserted inside the cable 13a. Moreover, a connection member (not shown) which is detachable from the light guide pipe sleeve 12 is provided at one end portion of the cable 13a. Also, a light guide connector 14, which is detachable from the light source apparatus 3, is provided at the other end portion of the cable 13a.

The illumination lens 15 configured to transmit light transmitted by the light guide 11 to outside, and an objective lens 17 configured to obtain an optical image according to light entering from outside are provided in the distal end portion of the insertion section 6. Also, an illumination window (not shown) where the illumination lens 15 is disposed, and an objective window (not shown) where the objective lens 17 is disposed are provided, adjacent to each other, at a distal end surface of the insertion section 6.

As shown in Fig. 2, a relay lens 18 provided with a plurality of lenses LE configured to transmit an optical image obtained by the objective lens 17 to the eyepiece section 7 is provided inside the insertion section 6. That is, the relay lens 18 includes a function of an optical transmission system configured to transmit light entering from the objective lens 17.

As shown in Fig. 2, an eyepiece lens 19 configured to allow observation, with a naked eye, of an optical image transmitted by the relay lens 18 is provided inside the eyepiece section 7.

The camera unit 22 includes an excitation light cut filter 23, an image pickup section 24, and a signal processing circuit 27.

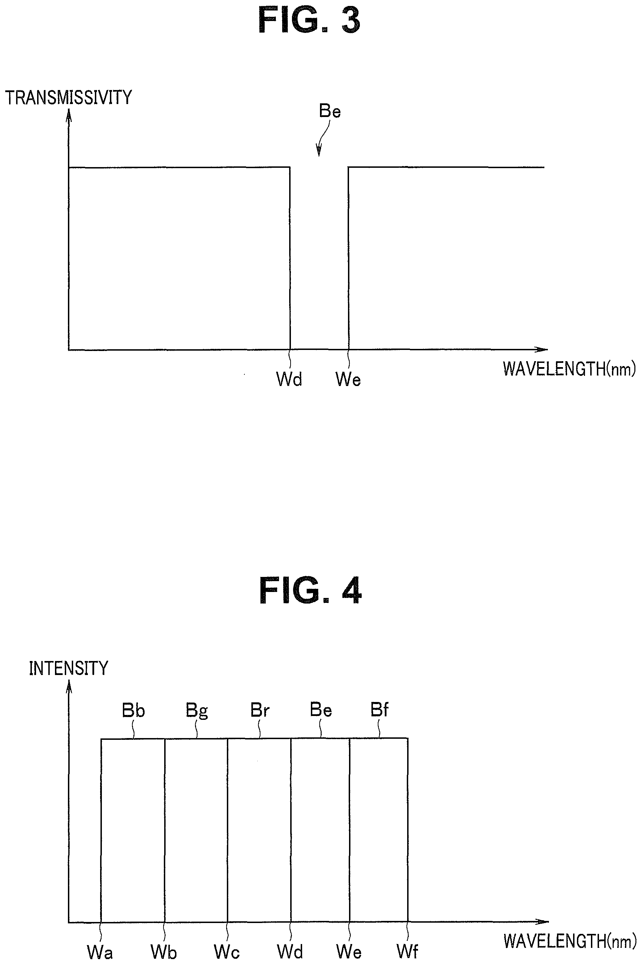

The excitation light cut filter 23 is disposed at a front of a dichroic prism 25, and is configured as an optical filter configured to remove reflected light of excitation light from light that is emitted through the eyepiece lens 19. More specifically, as shown in Fig. 3, the excitation light cut filter 23 is formed as an optical filter having optical characteristics of blocking light included in a wavelength band Be from a wavelength Wd, which belongs near to a boundary between a red range and a near-infrared range, to a wavelength We, which belongs to the near-infrared range and which is longer than the wavelength Wd, in light that is emitted through the eyepiece lens 19, and of transmitting light outside the wavelength band Be, for example. Fig. 3 is a diagram showing transmission characteristics as an example of optical characteristics of the excitation light cut filter provided in the endoscope apparatus according to the embodiment.

The image pickup section 24 includes the dichroic prism 25, and image pickup devices 26A, 26B and 26C.

The dichroic prism 25 is configured as a spectral element configured to separate light that is transmitted through the excitation light cut filter 23 into light in three wavelength bands of a red to near-infrared range, a green range and a blue range, and to output the light.

For example, the image pickup device 26A is configured by an image sensor such as a monochrome CCD. Also, the image pickup device 26A is configured to perform an image pickup operation according to an image pickup device drive signal outputted from the video processor 4. Moreover, the image pickup device 26A is configured to pick up light in the red to near-infrared range outputted through the dichroic prism 25, and to generate and output an image pickup signal according to the light in the red to near-infrared range which has been picked up.

For example, the image pickup device 26B is configured by an image sensor such as a monochrome CCD. Also, the image pickup device 26B is configured to perform an image pickup operation according to an image pickup device drive signal outputted from the video processor 4. Moreover, the image pickup device 26B is configured to pick up light in the green range outputted through the dichroic prism 25, and to generate and output an image pickup signal according to the light in the green range which has been picked up.

For example, the image pickup device 26C is configured by an image sensor such as a monochrome CCD. Also, the image pickup device 26C is configured to perform an image pickup operation according to an image pickup device drive signal outputted from the video processor 4. Moreover, the image pickup device 26C is configured to pick up light in the blue range outputted through the dichroic prism 25, and to generate and output an image pickup signal according to the light in the blue range which has been picked up.

That is, the image pickup section 24 is configured to be able to separate light that is emitted through the excitation light cut filter 23 into light in three wavelength bands of the red to near-infrared range, the green range, and the blue range, and to pick up the light.

Note that the image pickup section 24 according to the present embodiment is not limited to a configuration including the dichroic prism 25 and the image pickup devices 26A, 26B and 26C, and may alternatively be configured to include, at an image pickup surface, a color filter which is capable of separating light emitted through the excitation light cut filter 23 into light in three wavelength bands of the red to near-infrared range, the green range, and the blue range, and to include one image pickup device configured to pick up light received at the image pickup surface and to output an image pickup signal.

The signal processing circuit 27 generates image signals by subjecting image pickup signals outputted from the image pickup devices 26A, 26B and 26C to predetermined signal processing such as a correlated double sampling process and an A/D conversion process, and to output the generated image signals to the video processor 4 to which a signal cable 28 is connected.

The light source apparatus 3 is configured to be able to supply excitation light for generating fluorescence by exciting a fluorescent pigment administered to a subject, and reference light, which is visible light in a different wavelength band from the fluorescence. Moreover, the light source apparatus 3 includes a light emitting section 31, an optical deflection section 32, a condenser lens 33, a memory 34, and a light source control section 35.

The light emitting section 31 includes a red light source 31A, a green light source 31B, a blue light source 31C, and an excitation light source 31D.

For example, the red light source 31A includes a lamp or an LED, and is configured to emit R light (see Fig. 4), which is red light in a wavelength band Br from a wavelength Wc, which belongs near to a boundary between the green range and the red range and which is shorter than a wavelength Wd, to the wavelength Wd. Furthermore, the red light source 31A is configured to switch to an illuminated state or a non-illuminated state under the control of the light source control section 35. Moreover, in the illuminated state, the red light source 31A generates R light at an intensity or in an amount of light according to the control by the light source control section 35. Fig. 4 is a diagram showing an example of a wavelength band of light emitted from each light source provided in the light source apparatus according to the embodiment, and of a wavelength band of fluorescence that is emitted from a fluorescent pigment.

For example, the green light source 31B includes a lamp or an LED, and is configured to emit G light (see Fig. 4), which is green light in a wavelength band Bg from a wavelength Wb, which belongs near to a boundary between the blue range and the green range, to the wavelength Wc. Furthermore, the green light source 31B is configured to switch to an illuminated state or a non-illuminated state under the control of the light source control section 35. Moreover, in the illuminated state, the green light source 31B is configured to generate G light at an intensity or in an amount of light according to the control by the light source control section 35.

For example, the blue light source 31C includes a lamp or an LED, and is configured to emit B light (see Fig. 4), which is blue light in a wavelength band Bb from a wavelength Wa, which belongs to the blue range, to the wavelength Wb. Furthermore, the blue light source 31C is configured to switch to an illuminated state or a non-illuminated state under the control of the light source control section 35. Moreover, in the illuminated state, the blue light source 31C is configured to generate B light at an intensity or in an amount of light according to the control by the light source control section 35.

For example, the excitation light source 31D includes a lamp or an LED, and is configured to emit EX light (see Fig. 4), which is excitation light in a wavelength band Be from the wavelength Wd to the wavelength We. Furthermore, the excitation light source 31D is configured to switch to an illuminated state or a non-illuminated state under the control of the light source control section 35. Moreover, in the illuminated state, the excitation light source 31D is configured to generate EX light at an intensity or in an amount of light according to the control by the light source control section 35.

The multiplexer 32 is configured to be able to multiplex light emitted from the light emitting section 31 and to cause the light to enter the condenser lens 33.

The condenser lens 33 is configured to condense light entering through the optical deflection section 32, and to transmit the light to the light guide 13.

The memory 34 stores, as information unique to each light source apparatus 3, light source information, which is information allowing the type of light that is emitted from the light emitting section 31 to be separately identified, for example. The light source information stored in the memory 34 is read out by a control section 45 of the video processor 4 when the light source apparatus 3 and the video processor 4 are connected and the power of the video processor 4 is turned on.

The light source control section 35 is configured to control each light source of the light emitting section 31 based on an illumination control signal that is outputted from the video processor 4.

The video processor 4 includes an image pickup device drive section 41, an image processing section 42, an observation image generation section 43, an input I/F (interface) 44, and the control section 45.

For example, the image pickup device drive section 41 includes a driver circuit. Moreover, for example, the image pickup device drive section 41 is configured to generate and output an image pickup device drive signal for driving, for an exposure time according to control by the control section 45, each of the image pickup devices 26A, 26B and 26C.

For example, the image processing section 42 includes an image processing circuit. Moreover, the image processing section 42 is configured to subject an image signal outputted from the endoscope apparatus 2 to predetermined image processing such as tone correction processing, under the control of the control section 45, and to output the signal.

For example, the observation image generation section 43 includes an RGB signal generation circuit configured to generate an RGB signal that is used at the time of image display at the display apparatus 5. Moreover, the observation image generation section 43 is configured to generate an observation image by using an image signal that is outputted via the image processing section 42, according to the control of the control section 45, and to output the observation image. More specifically, the observation image generation section 43 generates an observation image by selectively assigning image signals outputted via the image processing section 42 to an R channel corresponding to a red component of the display apparatus 5, a G channel corresponding to a green component of the display apparatus 5, and a B channel corresponding to a blue component of the display apparatus 5, under the control of the control section 45, and outputs the generated observation image to the display apparatus 5.

The input I/F 44 includes one or more switches and/or buttons enabling instruction according to a user operation. More specifically, the input I/F 44 includes an observation mode switching switch (not shown) which is capable of issuing an instruction according to a user operation so as to set (switch) the observation mode of the endoscope system 1 to one of a white light observation mode and a fluorescence observation mode, for example.

For example, the control section 45 includes a control circuit such as a CPU or an FPGA (field programmable gate array). Moreover, the control section 45 is configured to detect the type of light that can be emitted from the light source apparatus 3, based on the light source information read from the memory 34. Also, the control section 45 is configured to generate, and to output to the light source control section 35, an illumination control signal for causing light according to a set observation mode to be emitted, based on the observation mode set by the observation mode switching switch of the input I/F 44 and the detection result of the type of light that can be emitted from the light source apparatus 3. Furthermore, the control section 45 is configured to control the image pickup device drive section 41 and the image processing section 42 such that an operation according to the observation mode set by the observation mode switching switch of the input I/F 44 is performed. Moreover, the control section 45 controls the observation image generation section 43 so as to assign an image signal outputted via the image processing section 42 to each color channel of the display apparatus 5 such that an observation image according to the observation mode set by the observation mode switching switch of the input I/F 44 is generated.

For example, the display apparatus 5 includes an LCD (liquid crystal display), and is configured to be able to display an observation image that is outputted from the video processor 4.

Next, an operation and the like of the endoscope system 1 of the present embodiment will be described. Note that in the present embodiment, description is given by citing, as an example, a case where a fluorescent pigment FLP having fluorescence characteristics according to which FL light (see Fig. 4), which is fluorescence (near-infrared light) in a wavelength band Bf from the wavelength We to a wavelength Wf, which belongs to the near-infrared range and which is longer than the wavelength We, is emitted according to radiation of EX light.

First, a user such as a surgeon connects each section of the endoscope system 1 and turns on the power, and then, issues an instruction by operating the input I/F 44 so as to set the observation mode of the endoscope system 1 to the white light observation mode.

When the light source apparatus 3 and the video processor 4 are connected to each other, and the power of the video processor 4 is turned on, the control section 45 reads the light source information stored in the memory 34, and detects, based on the light source information read out, four types of light, R light, G light, B light and EX light, as the types of light that can be emitted from the light source apparatus 3. Then, when setting of the white light observation mode is detected, the control section 45 generates, and outputs to the light source control section 35, an illumination control signal for causing the R light, the G light and the B light, among the four types of light detected based on the light source information read from the memory 34, to be simultaneously emitted from the light source apparatus 3.

When the white light observation mode is set, the light source control section 35 performs control so as to place the red light source 31A, the green light source 31B, and the blue light source 31C in the illuminated state, according to the illumination control signal outputted from the control section 45, and also performs control to place the excitation light source 31D in the non-illuminated state.

Furthermore, when the operation as described above is performed by the light source control section 35, WL light, which is white light including the R light, the G light and the B light, is radiated on a target, and WLR light, which is reflected light generated by the target according to radiation of the WL light, enters from the objective lens 17 as return light. Also, the WLR light entering from the objective lens 17 is emitted to the camera unit 22 through the relay lens 18 and the eyepiece lens 19.

According to the configuration of the camera unit 22 described above, because the excitation light cut filter 23 has the optical characteristics as shown in Fig. 3, the WLR light emitted through the eyepiece lens 19 is transmitted through the excitation light cut filter 23 to enter the dichroic prism 25. Then, the dichroic prism 25 separates the WLR light, which is incident light, into R light, G light and B light, and the light is emitted.

Accordingly, when the observation mode of the endoscope system 1 is set to the white light observation mode, the R light included in WLR light is picked up by the image pickup device 26A, the G light included in the WLR light is picked up by the image pickup device 26B, and the B light included in the WLR light is picked up by the image pickup device 26C. Also, when the observation mode of the endoscope system 1 is set to the white light observation mode, an image signal RS corresponding to the image pickup signal outputted from the image pickup device 26A, an image signal GS corresponding to the image pickup signal outputted from the image pickup device 26B, and an image signal BS corresponding to the image pickup signal outputted from the image pickup device 26C are generated by the signal processing circuit 27, and also, the generated image signals RS, GS and BS are outputted from the signal processing circuit 27 to the observation image generation section 43 via the image processing section 42.

Moreover, when setting of the white light observation mode is detected, the control section 45 controls the observation image generation section 43 such that the image signal outputted via the image processing section 42 is assigned to each color channel of the display apparatus 5 so that an observation image according to the return light picked up by the image pickup devices 26A, 26B and 26C is generated in the white light observation mode.

More specifically, for example, the control section 45 controls the observation image generation section 43 such that an observation image to be displayed by the display apparatus 5 is generated, by causing, among image signals outputted via the image processing section 42, the image signal RS obtained by picking up the R light by the image pickup device 26A to be assigned to the R channel of the display apparatus 5, the image signal GS obtained by picking up the G light by the image pickup device 26B to be assigned to the G channel of the display apparatus 5, and the image signal BS obtained by picking up the B light by the image pickup device 26C to be assigned to the B channel of the display apparatus 5.

When the white light observation mode is set, the observation image generation section 43 generates a white light observation image by assigning the image signal RS outputted via the image processing section 42 to the R channel of the display apparatus 5, the image signal GS outputted via the image processing section 42 to the G channel of the display apparatus 5, and the image signal BS outputted via the image processing section 42 to the B channel of the display apparatus 5, according to control of the control section 45, and outputs the generated white light observation image to the display apparatus 5.

According to the operations of the observation image generation section 43 and the control section 45 as described above, a white light observation image having substantially the same color tone as when a target such as a living tissue is seen with a naked eye is displayed by the display apparatus 5, for example.

On the other hand, the user administers the fluorescent pigment FLP to a subject at a desired timing before the observation mode of the endoscope system 1 is set to the fluorescence observation mode, for example.

Moreover, for example, the user operates the input I/F 44 at a desired timing before the observation mode of the endoscope system 1 is set to the fluorescence observation mode, to thereby input information about the fluorescence characteristics of the fluorescent pigment FLP to be administered to the subject. Then, based on such an input operation of the user, the control section 45 can recognize that the return light that is generated according to radiation of the EX light is FL light in the wavelength band Bf.

The user inserts the insertion section 6 into the subject while checking the white light observation image displayed by the display apparatus 5, and operates the input I/F 44 in a state where the distal end portion of the insertion section 6 is arranged near a desired observation position inside the subject, and thereby issues an instruction for setting the observation mode of the endoscope system 1 to the fluorescence observation mode.

When setting of the fluorescence observation mode is detected, the control section 45 generates, and outputs to the light source control section 35, an illumination control signal for causing the G light, the B light and the EX light, among the four types of light detected based on the light source information read from the memory 34, to be simultaneously emitted from the light source apparatus 3.

When the fluorescence observation mode is set, the light source control section 35 performs control so as to place the green light source 31B, the blue light source 31C, and the excitation light source 31D in the illuminated state, according to the illumination control signal outputted from the control section 45, and also performs control to place the red light source 31A in the non-illuminated state.

Furthermore, when the operation as described above is performed by the light source control section 35, EX light, which is the excitation light, and RF light, which is reference light including the G light and the B light, are radiated on the target, and FL light, which is emitted from the fluorescent pigment FLP according to radiation of the EX light, RL light, which is reflected light generated by the target according to radiation of the RF light, and EXR light, which is reflected light generated by the target according to radiation of the EX light, enter from the objective lens 17 as return light. Also, each light included in the return light entering from the objective lens 17 is emitted to the camera unit 22 through the relay lens 18 and the eyepiece lens 19.

According to the configuration of the camera unit 22 described above, because the excitation light cut filter 23 has the optical characteristics as shown in Fig. 3, the EXR light emitted through the eyepiece lens 19 is blocked by the excitation light cut filter 23, and the FL light and the RL light emitted through the eyepiece lens 19 are transmitted through the excitation light cut filter 23 to enter the dichroic prism 25. Then, the dichroic prism 25 separates the FL light and the RL light included in the incident light, and further separates the RL light into G light and B light, and the light is emitted.

Accordingly, when the observation mode of the endoscope system 1 is set to the fluorescence observation mode, the FL light is picked up by the image pickup device 26A, the G light included in the RL light is picked up by the image pickup device 26B, and the B light included in the RL light is picked up by the image pickup device 26C. Also, when the observation mode of the endoscope system 1 is set to the fluorescence observation mode, an image signal FS corresponding to the image pickup signal outputted from the image pickup device 26A, an image signal GS corresponding to the image pickup signal outputted from the image pickup device 26B, and an image signal BS corresponding to the image pickup signal outputted from the image pickup device 26C are generated by the signal processing circuit 27, and also, the generated image signals FS, GS and BS are outputted from the signal processing circuit 27 to the observation image generation section 43 via the image processing section 42.

Moreover, when setting of the fluorescence observation mode is detected, the control section 45 controls the observation image generation section 43 such that the image signal outputted via the image processing section 42 is assigned to each color channel of the display apparatus 5 so that an observation image according to the return light picked up by the image pickup devices 26A, 26B and 26C is generated in the fluorescence observation mode.

More specifically, for example, the control section 45 controls the observation image generation section 43 such that an observation image to be displayed by the display apparatus 5 is generated, by causing the image signal FS outputted via the image processing section 42 to be assigned to the G channel of the display apparatus 5, and by causing the image signal GS having a relatively large signal value, between the image signals GS and BS outputted via the image processing section 42, to be assigned to the R channel of the display apparatus 5 and the image signal BS having a relatively small signal value to be assigned to the B channel of the display apparatus 5.

When the fluorescence observation mode is set, the observation image generation section 43 generates a fluorescence observation image by assigning the image signal GS outputted via the image processing section 42 to the R channel of the display apparatus 5, the image signal FS outputted via the image processing section 42 to the G channel of the display apparatus 5, and the image signal BS outputted via the image processing section 42 to the B channel of the display apparatus 5, according to the control of the control section 45, and outputs the generated fluorescence observation image to the display apparatus 5.

For example, when taking into account that the peak of sensitivity of a human eye (photopic luminous efficiency function) is in the green range, visibility of a generated position of FL light, which is to be focused on in the fluorescence observation, is considered to be increased when the FL light is displayed in green on the screen of the display apparatus 5. Also, when taking into account that red is at a position farthest from green in a Lab color space, visibility of a peripheral region of a generated position of FL light, which is displayed in green on the screen of the display apparatus 5, from the generated position of the FL light is assumed to be ensured by displaying one of the G light and the B light, included in the RL light, with a relatively high amount of light in red on the screen of the display apparatus 5 and displaying the other light with a relatively low amount of light in blue on the screen of the display apparatus 5.

Accordingly, with the fluorescence observation image that is generated by the operations of the control section 45 and the observation image generation section 43 as described above, a fluorescence observation image with color tones allowing a generated position of FL light and a peripheral region of the generated position of the FL light to be easily and individually viewed is displayed by the display apparatus 5.

Note that the assignment destinations of the image signals GS and BS at the time of generation of the fluorescence observation image described above are set based on the characteristics of a living tissue that absorbance of light is reduced according to the wavelength of light in a visible range, and are thus applied in the case where the amount of G light and the amount of B light included in the reference light supplied from the light source apparatus 3 are the same or are substantially the same, that is, in the case where the signal value of the image signal GS is known to be constantly larger than the signal value of the image signal BS. Accordingly, in the fluorescence observation mode, for example, in the case of a situation where the signal value of the image signal BS is made larger than the signal value of the image signal GS by a light dimming operation of increasing the amount of B light included in reference light supplied from the light source apparatus 3 than the amount of G light included in the reference light, the control section 45 of the present embodiment may control the observation image generation section 43 such that the image signal BS outputted via the image processing section 42 is assigned to the R channel of the display apparatus 5 and the image signal GS outputted via the image processing section 42 is assigned to the B channel of the display apparatus 5.

That is, the control section 45 of the present embodiment may set image signals to be assigned to the R channel and the B channel of the display apparatus 5 in the fluorescence observation mode based only on each wavelength band of light included in the reference light supplied from the light source apparatus 3, or based on the combination of each wavelength band included in the reference light supplied from the light source apparatus 3 and the amount of light.

As described above, according to the present embodiment, when the fluorescence observation mode is set, a fluorescence observation image having color tones allowing a generated position of fluorescence and a peripheral region of the generated position of the fluorescence to be easily and individually viewed may be generated and displayed by the display apparatus 5. Therefore, according to the present embodiment, the diagnostic performance at the time of performing diagnosis of a desired observation position may be increased in fluorescence observation.

Note that the control section 45 of the present embodiment is not limited to performing control as described above, and may also control the observation image generation section 43 such that an observation image to be displayed by the display apparatus 5 is generated, by causing the image signal FS outputted via the image processing section 42 to be assigned to the G channel of the display apparatus 5, and causing the image signal GS having a relatively large signal value, between the image signals GS and BS outputted via the image processing section 42, to be assigned to the R channel of the display apparatus 5 and the image signal BS having a relatively small signal value to be assigned to the B channel and the G channel of the display apparatus 5.

Moreover, according to control by the control section 45 according to the modification described above, a fluorescence observation image in which a difference in chroma between a generated position of FL light and a peripheral region of the generated position of the FL light is emphasized may be generated and displayed by the display apparatus 5. Therefore, according to control by the control section 45 according to the modification described above, for example, even in a case where a difference in brightness or luminance between a generated position of FL light and a peripheral region of the generated position of the FL light is extremely small, the diagnostic performance at the time of performing diagnosis of a desired observation position may be increased in fluorescence observation.

Moreover, in addition to the control according to the modification described above, the control section 45 of the present embodiment may control the observation image generation section 43 in terms of gain adjustment of making a gain of the signal value of the image signal BS to be assigned to the G channel of the display apparatus 5 and a gain of the signal value of the image signal BS to be assigned to the B channel of the display apparatus 5 different from each other. More specifically, for example, in addition to the control according to the modification described above, the control section 45 may control the observation image generation section 43 in terms of gain adjustment of making a gain of the signal value of the image signal BS to be assigned to the G channel of the display apparatus 5 less than one times, and of making a gain of the signal value of the image signal BS to be assigned to the B channel of the display apparatus 5 one or more times.

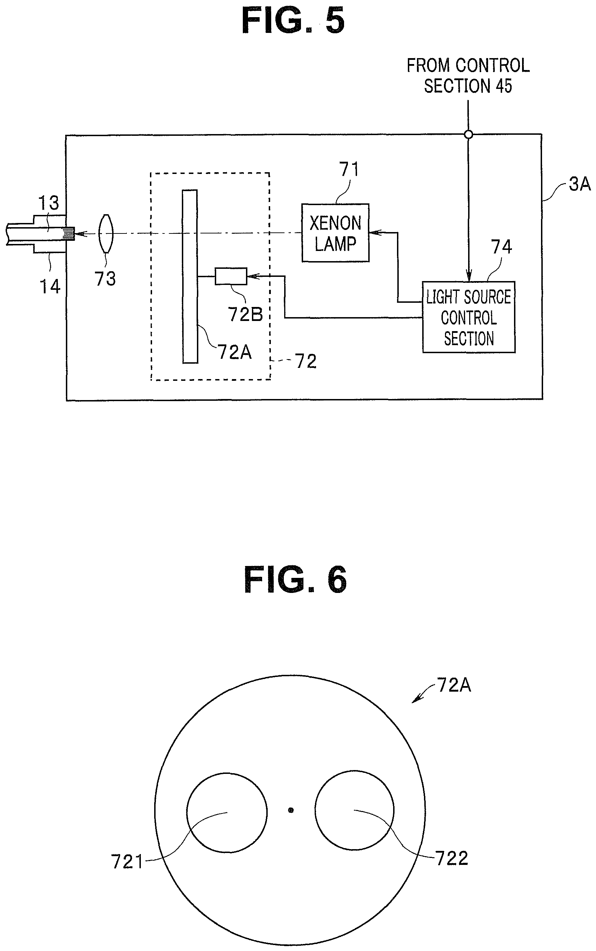

According to the present embodiment, the endoscope system 1 may be configured by using a light source apparatus 3A having a configuration as shown in Fig. 5, for example, instead of the light source apparatus 3. Fig. 5 is a diagram showing an example configuration of the light source apparatus according to the embodiment, different from the configuration shown in Fig. 2.

As shown in Fig. 5, the light source apparatus 3A includes a xenon lamp 71, a filter switching device 72, a condenser lens 73, and a light source control section 74.

For example, the xenon lamp 71 is configured to emit BL light, which is broadband light having a bandwidth from the wavelength Wa to the wavelength We. Moreover, the xenon lamp 71 is configured to switch to an illuminated state or a non-illuminated state under the control of the light source control section 74. Moreover, in the illuminated state, the xenon lamp 71 generates BL light in an amount of light according to the control by the light source control section 74.

The filter switching device 72 includes a rotation filter 72A, which is provided so as to perpendicularly cross an optical path of light emitted by the xenon lamp 71, and a motor 72B configured to switch a filter to be interposed on the optical path of light emitted from the xenon lamp 71 to one of filters of the rotation filter 72A by being rotated according to control by the light source control section 74.

For example, the rotation filter 72A is formed into a disk shape. Also, as shown in Fig. 6, the rotation filter 72A is provided with a white light observation filter 721 and a fluorescence observation filter 722, for example. Fig. 6 is a diagram showing an example configuration of the rotation filter provided in the light source apparatus in Fig. 5.

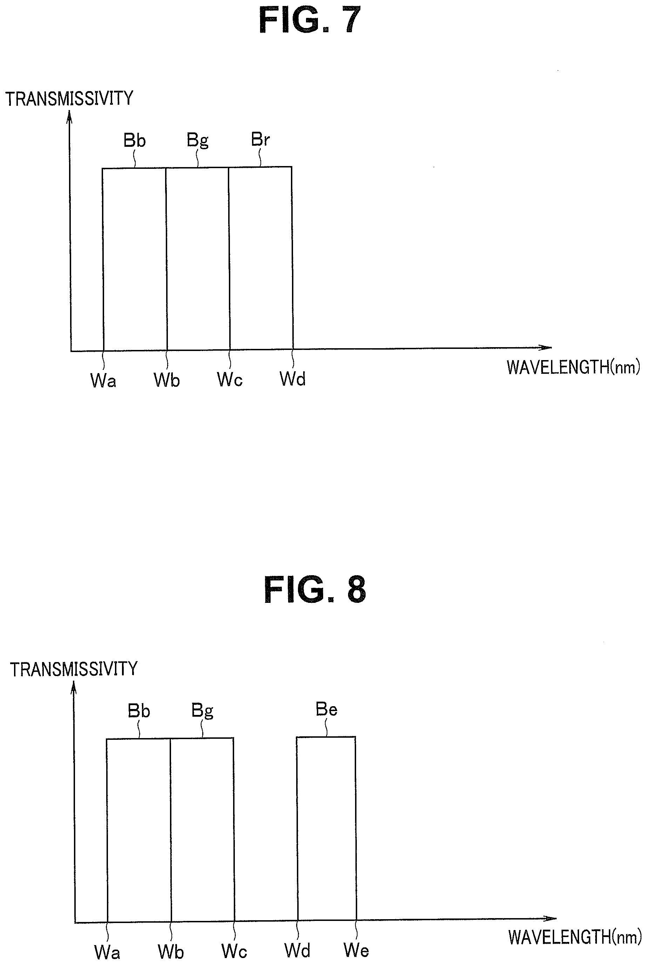

For example, as shown in Fig. 7, the white light observation filter 721 is formed to have optical characteristics of transmitting light included in any of three wavelength bands, a wavelength band Br, a wavelength band Bg and a wavelength band Bb, and of blocking light included in wavelength bands other than the three wavelength bands. Fig. 7 is a diagram showing an example of transmission characteristics of the white light observation filter provided in the rotation filter in Fig. 6.

For example, as shown in Fig. 8, the fluorescence observation filter 722 is formed to have optical characteristics of transmitting light included in any of three wavelength bands, the wavelength band Bg, the wavelength band Bb and a wavelength band Be, and of blocking light included in wavelength bands other than the three wavelength bands. Fig. 8 is a diagram showing an example of transmission characteristics of the fluorescence observation filter provided in the rotation filter in Fig. 6.

That is, the filter switching device 72 is configured to be able to interpose one of the white light observation filter 721 and the fluorescence observation filter 722 on the optical path of light emitted from the xenon lamp 71, and to retract the other filter different from the one filter from the optical path, by rotating the motor 72B according to the control by the light source control circuit 74.

The condenser lens 73 is configured to condense light entering via the filter switching device 72, and to transmit the light to the light guide 13.

The light source control section 74 is configured to control the xenon lamp 71 and the filter switching device 72 based on illumination control signals outputted from the control section 45 of the video processor 4.

More specifically, for example, when setting of the observation mode of the endoscope system 1 to the white light observation mode is detected, the light source control section 74 controls the xenon lamp 71 such that a predetermined amount of BL light is generated, and controls the motor 72B of the filter switching device 72 such that the white light observation filter 721 is interposed on the optical path of the light to be emitted from the xenon lamp 71, based on illumination control signals outputted from the control section 45. Also, for example, when setting of the observation mode of the endoscope system 1 to the fluorescence observation mode is detected, the light source control section 74 controls the xenon lamp 71 such that a predetermined amount of BL light is generated, and controls the motor 72B of the filter switching device 72 such that the fluorescence observation filter 722 is interposed on the optical path of the light to be emitted from the xenon lamp 71, based on illumination control signals outputted from the control section 45.

Furthermore, according to the present embodiment, the same effects as described above may be achieved in the case where the endoscope system 1 is configured by using the light source apparatus 3A having the configuration described above, instead of the light source apparatus 3.

Moreover, the embodiment described above is not only applied to the endoscope apparatus 2 including the telescope 21 and the camera unit 22, but also may be applied, in substantially the same manner, to an electronic endoscope including an elongated insertion section that can be inserted into a subject, and an image pickup section 24 provided at a distal end portion of the insertion section and configured to pick up an image of a target inside the subject.

According to the present embodiment, when the observation mode of the endoscope system 1 is set to the fluorescence observation mode, selection between a first display mode in which the fluorescence observation image, described above, generated by using the image signals FS, GS and BS is caused to be displayed by the display apparatus 5, and a second display mode in which a fluorescence observation image generated by using only the image signal FS is caused to be displayed by the display apparatus 5 may be performed according to operation of the input I/F 44, for example. Also, according to such a configuration, to increase the visibility of a generation state of FL light in the second display mode, the control section 45 may perform control to set the exposure time of the image pickup device 26A when the second display mode is selected to be longer than the exposure time of the image pickup device 26A when the first display mode is selected, for example.

Note that the present invention is not limited to the embodiment and the modifications described above, and various changes and applications may, of course, be made within the scope of the invention.

The present application claims priority from Japanese Patent Application No. 2015-208029 filed in Japan on October 22, 2015, the entire contents of which are incorporated in the present description and claims by reference.

## Claims

1. An endoscope system comprising:
a light source apparatus capable of supplying excitation light for exciting a fluorescent pigment administered to a subject and for generating fluorescence, and reference light that is visible light in a different wavelength band from the fluorescence;
an objective optical system, provided in a distal end portion of an insertion section that can be inserted into the subject, to which the fluorescence, reflected light of the reference light generated according to radiation of the reference light, and reflected light of the excitation light generated according to radiation of the excitation light enter as return light;
an optical filter including optical characteristics of transmitting the fluorescence and the reflected light of the reference light included in the return light entering from the objective optical system, and of blocking the reflected light of the excitation light included in the return light entering from the objective optical system;
an image pickup section configured to be able to separate, and pick up, light that is emitted through the optical filter into the fluorescence, light in a first wavelength band included in the reflected light of the reference light, and light in a second wavelength band, different from the light in the first wavelength band, included in the reflected light of the reference light;
an observation image generation section configured to generate an observation image using a first image signal that is obtained by picking up the fluorescence, a second image signal that is obtained by picking up the light in the first wavelength band, and a third image signal that is obtained by picking up the light in the second wavelength band, and to output the observation image to a display apparatus; and
a control section configured to control the observation image generation section such that the observation image is generated, by causing the first image signal to be assigned to a first channel corresponding to a green component of the display apparatus, and by causing one image signal having a relatively large signal value, between the second image signal and the third image signal, to be assigned to a second channel corresponding to a red component of the display apparatus and another image signal having a relatively small signal value to be assigned to a third channel corresponding to a blue component of the display apparatus.

2. The endoscope system according to claim 1, wherein the image pickup section includes
a dichroic prism configured to separate, and transmit, light that is transmitted through the optical filter into the fluorescence, the light in the first wavelength band, and the light in the second wavelength band, and
three image pickup devices configured to pick up, respectively, the fluorescence, the light in the first wavelength band, and the light in the second wavelength band that are light emitted through the dichroic prism.

3. The endoscope system according to claim 1, wherein in a case where the fluorescence is near-infrared light, the light in the first wavelength band is green light, and the light in the second wavelength band is blue light, the control section controls the observation image generation section such that the observation image is generated, by causing the first image signal to be assigned to the first channel, causing the second image signal to be assigned to the second channel, and causing the third image signal to be assigned to the third channel.

4. An endoscope system comprising:
a light source apparatus capable of supplying excitation light for exciting a fluorescent pigment administered to a subject and for generating fluorescence, and reference light that is visible light in a different wavelength band from the fluorescence;
an objective optical system, provided in a distal end portion of an insertion section that can be inserted into the subject, to which the fluorescence, reflected light of the reference light generated according to radiation of the reference light, and reflected light of the excitation light generated according to radiation of the excitation light enter as return light;
an optical filter including optical characteristics of transmitting the fluorescence and the reflected light of the reference light included in the return light entering from the objective optical system, and of blocking the reflected light of the excitation light included in the return light entering from the objective optical system;
an image pickup section configured to be able to separate, and pick up, light that is emitted through the optical filter into the fluorescence, light in a first wavelength band included in the reflected light of the reference light, and light in a second wavelength band, different from the light in the first wavelength band, included in the reflected light of the reference light;
an observation image generation section configured to generate an observation image using a first image signal that is obtained by picking up the fluorescence, a second image signal that is obtained by picking up the light in the first wavelength band, and a third image signal that is obtained by picking up the light in the second wavelength band, and to output the observation image to a display apparatus; and
a control section configured to control the observation image generation section such that the observation image is generated, by causing the first image signal to be assigned to a first channel corresponding to a green component of the display apparatus, and by causing one image signal having a relatively large signal value, between the second image signal and the third image signal, to be assigned to a second channel corresponding to a red component of the display apparatus and another image signal having a relatively small signal value to be assigned to a third channel corresponding to a blue component of the display apparatus and the first channel.

5. The endoscope system according to claim 4, wherein the image pickup section includes
a dichroic prism configured to separate, and transmit, light that is transmitted through the optical filter into the fluorescence, the light in the first wavelength band, and the light in the second wavelength band, and
three image pickup devices configured to pick up, respectively, the fluorescence, the light in the first wavelength band, and the light in the second wavelength band that are light emitted through the dichroic prism.

6. The endoscope system according to claim 4, wherein in a case where the fluorescence is near-infrared light, the light in the first wavelength band is green light, and the light in the second wavelength band is blue light, the control section controls the observation image generation section such that the observation image is generated, by causing the first image signal to be assigned to the first channel, causing the second image signal to be assigned to the second channel, and causing the third image signal to be assigned to the third channel and the first channel.
